(19) Europäisches Patentamt
European Patent Office
Office européen des brevets

(11) Veröffentlichungsnummer : **0 431 450 B1**

# (12) EUROPÄISCHE PATENTSCHRIFT

(45) Veröffentlichungstag der Patentschrift :
**13.04.94 Patentblatt 94/15**

(51) Int. Cl.[5] : **C07C 33/46,** C07C 29/62, C07D 405/06

(21) Anmeldenummer : **90122697.7**

(22) Anmeldetag : **28.11.90**

## (54) 1-Brom-3-chlor-1,2-diaryl-2-propanole, Verfahren zur stereoselektiven Herstellung der erythro-1-Brom-3-chlor-1,2-diaryl-2-propanole und deren Umsetzung zu Azolylmethyloxiranen.

(30) Priorität : **07.12.89 DE 3940492**

(43) Veröffentlichungstag der Anmeldung :
**12.06.91 Patentblatt 91/24**

(45) Bekanntmachung des Hinweises auf die Patenterteilung :
**13.04.94 Patentblatt 94/15**

(84) Benannte Vertragsstaaten :
**AT BE CH DE DK ES FR GB GR IT LI NL SE**

(56) Entgegenhaltungen :
**Keine einschlägigen Dokumente gefunden**

(73) Patentinhaber : **BASF Aktiengesellschaft**
**Carl-Bosch-Strasse 38**
**D-67063 Ludwigshafen (DE)**

(72) Erfinder : **Kober, Reiner, Dr.**
**Im Schlittweg 20**
**W-6701 Fussgoenheim (DE)**
Erfinder : **Isak, Heinz, Dr.**
**Schoenfelderstrasse 3**
**W-6704 Mutterstadt (DE)**
Erfinder : **Seele, Rainer, Dr.**
**Leiblstrasse 3**
**W-6701 Fussgoenheim (DE)**

EP 0 431 450 B1

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die vorliegende Erfindung betrifft 1-Brom-3-chlor-1,2-diaryl-2-propanole der allgemeinen Formel I

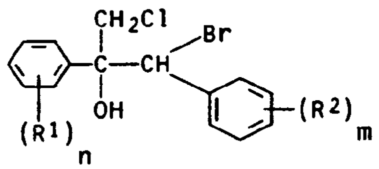

(I),

in welcher n und m für 1, 2 oder 3 stehen und die Substituenten $R^1$ und $R^2$ unabhängig voneinander die folgende Bedeutung haben:

Wasserstoff, Halogen, eine $C_1$-$C_6$-Halogenalkylgruppe, eine $C_1$-$C_5$-Alkoxygruppe, eine $C_1$-$C_5$-Halogenalkoxygruppe, t-Butyl oder einen aromatischen Rest, der unsubstituiert oder ein- bis dreifach durch Halogen, eine $C_1$-$C_5$-Alkoxygruppe, eine $C_1$-$C_5$-Halogenalkoxygruppe oder t-Butyl substituiert ist.

Weiterhin betrifft die Erfindung die Herstellung der Verbindungen I, insbesondere die stereoselektive Herstellung der erythro-konfigurierten Verbindungen I und deren Umsetzung zu Azolylmethyloxiranen der Formel III

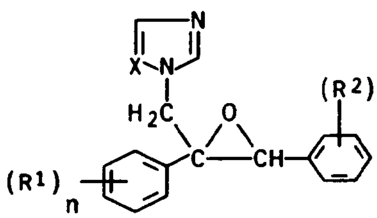

(III),

in der die Reste die o.g. Bedeutung haben und X für CH oder N steht.

Aus den deutschen Offenlegungsschriften DE-A 32 18 129 und 33 18 130 geht hervor, daß die Azolylmethyloxirane III eine gute antimykotische und fungizide Wirksamkeit aufweisen, wobei insbesondere die Z- oder cis-Oxirane gemäß der EP-A 196 038 eine ausgeprägte fungizide Aktivität zeigen.

Der Erfindung lag daher die Aufgabe zugrunde, cis-konfigurierte Azolylmethyloxirane des Strukturtyps III in einer möglichst kurzen Synthesesequenz und in einer hoher Ausbeute herzustellen, wobei die bislang übliche Verwendung von Peroxidverbindungen zur Epoxidation vermieden werden sollte.

Es wurde nun gefunden, daß die eingangs definierten 1-Brom-3-chlor-1,2-diaryl-2-propanole I einen vorteilhaften Zugang zu den Azolylmethyloxiranen III ermöglichen.

Zur regioselektiven Herstellung der cis-konfigurierten Azolylmethyloxirane sind insbesondere die erythro-konfigurierten Propanole I und deren optische Antipoden, die durch die Fischer-Projektionen Ia und Ib, worin Ar für Aryl steht, wiedergegeben werden:

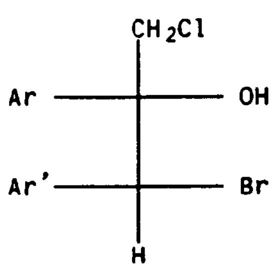

(Ia)

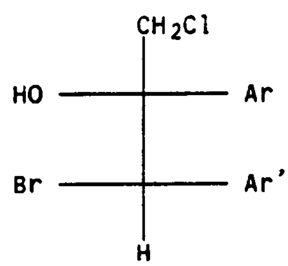

(Ib)

geeignet.

Die Herstellung der 1-Brom-3-chlor-1,2-diaryl-2-propanole I erfolgt erfindungsgemäß durch radikalische Bromierung von Chlorhydrinen der allgemeinen Formel II

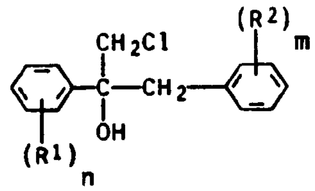

(II),

in der die Reste $(R^1)_n$ und $(R^2)_m$ die für Verbindung I genannte Bedeutung haben, in einem inerten aprotischen Lösungsmittel.

Im Hinblick auf den Stand der Technik, z.B. Earl S. Huyser in Free-Radical Chain Reaktions, Wiley-Interscience, 1970, Seiten 90 bis 103, wurde überraschend gefunden, daß durch die radikalische Bromierung stereoselektiv das gewünschte erythro-konfigurierte Propanol I gebildet wird, wobei man erythro/threo-Anteile (s. Formeln Ia und Ib) von 3:1 bis 7:1 findet.

Eine Umlagerungsreaktion des intermediär entstehenden β-Phenylalkyl-Radikals unter Wanderung des $(R^1)_n$-substituierten Arylrestes oder die Arylumlagerung an einem möglichen tert-Alkoxy-Radikal (s. loc. cit, Seite 253) wird praktisch überraschenderweise nicht gefunden. Ebenso findet man praktisch keine Wasserabspaltung zu den nach DE-A 32 10 570 bekannten Diaryl-dihalogen-propenen.

Für die radikalische Bromierung können dazu übliche Bromierungsreagenzien wie N-Bromverbindungen von Amiden oder Imiden verwendet werden. Beispielsweise seien N-Bromsuccinimid, 1,3-Dibrom-5,5-dimethylhydantoin oder N-Bromphthalimid genannt. Ferner können elementares Brom, Bromtrichlormethan, Brompentachlorethan oder tert-Butylhypobromit verwendet werden. In manchen Fällen kann es vorteilhaft sein, katalytische Mengen an Aktivatoren wie z.B. Jodbenzol, Kobalt- oder Kupfer-II-Salze wie $CoBr_3$, $CuBr_2$, $PCl_3$, $PBr_3$ oder quartäre Ammoniumbromide, wie z.B. N-Tetrabutylammonium-bromid zur Beschleunigung der Reaktion hinzuzusetzen.

Die Menge der Bromierungsreagenzien liegt im allgemeinen bei 1 bis 5, insbesondere 1 bis 2 Moläquivalenten, je Mol Chlorhydrin II.

Die Bromierung kann bei Reaktionstemperaturen von 0 bis 100°C, bevorzugt 10 bis 80°C, durchgeführt werden.

Bei niedrigen Temperaturen, z.B. Temperaturen unter 50°C kann die Radikalkettenreaktion in für Bromierungsreaktionen bekannter Weise durch gleichzeitiges Bestrahlen des Reaktionsgemisches mit einer Photolampe vorgenommen werden. Dabei kann es vorteilhaft sein, gleichzeitig katalytische Mengen an bekannten Radikalstartern hinzuzugeben, die zusätzlich durch photochemische Spaltung den Radikalkettenprozeß in Gang setzten. Geeignete Photolampen sind z.B. in Houben-Weyl, Methoden der org. Chemie, Band 4/5a, Seite 143ff. beschrieben.

Bei Temperaturen über ca. 50°C läßt sich die Reaktion unter Umständen auch ohne zusätzliches Bestrahlen nur mit Radikalstartern zur Initiirung der Radikalreaktion durchführen.

Als Radikalstarter können dazu übliche Verbindungen des Azo- oder Peroxidtyps wie tert.-Butylperoxyneodecanoat, Diacetylperoxid, Bis-(2,4-dichlor-benzoyl)-peroxid, Diisopropylperoxydicarbonat, Dicyclohexylperoxydicarbonat, Acetylcyclohexansulfonylperoxid, Dibenzoylperoxid, Dipropionylperoxid, Di-sek.-butyl-peroxydicarbonat, Di-tert.-butylperoxydicarbonat, Di-(4-tert.-butyl-cyclohexyl)-peroxydicarbonat sowie Azo-bis-isobutyronitril.

Die Menge des Radikalstarters liegt im allgemeinen bei 0,5 bis 15 Mol.-%, bezogen auf das Chlorhydrin II. Höhere Mengen sind möglich, aber in der Regel nicht erforderlich.

Typische Reaktionstemperaturen für die Bromierung sind solche Temperaturen, bei denen die Radikalstarter eine Halbwertszeit von einer Stunde aufweisen, i.a. ist das bei ca. 30 bis 80°C.

Als aprotische Lösungsmittel für die radikalische Bromierung können die dazu üblichen z.B. in Houben-Weyl, Methoden der organischen Chemie, Band 4/5a, Seiten 143ff. genannten Lösungsmittel verwendet werden. Derartige Lösungsmittel sind z.B. halogenierte aliphatische oder aromatische Kohlenwasserstoffe, wie Methylenchlorid, Tetrachlormethan, Chlorbenzol, 1,2-Dichlorethan, Trichlorethylen, Hexafluorethan. Ferner Fluor/Chlor-Kohlenwasserstoffe wie 1, 1-Difluor-1,2, 2, 2-tetrachlorethan, 1,1,1-Trifluor-2, 2, 2-trichlorethan oder fluorierte Kohlenwasserstoffe, wie Fluorbenzol oder Trifluormethylbenzol.

Neben diesen bevorzugten Lösungsmitteln können auch Kohlenwasserstoffe wie Cyclohexan, n-Pentan, n-Hexan, Benzol; Nitrobenzol oder Nitrile wie Propionitril, Acetonitril sowie Schwefelkohlenstoff verwendet werden.

Die Menge des Lösungmittels ist nicht besonders kritisch. In der Regel wird auf 2 bis 20 gew.-%ige Lösungen der eingesetzten Chlorhydrine der Formel II verdünnt.

Die Chlorhydrine der allgemeinen Formel II sind bekannte Verbindungen, die durch Benzyl-Grignard-Reaktion an ω-Chloracetophenone, z.B. wie in DE-A 29 20 374, EP-B 15 756, DE-A 28 51 086 oder EP-A 47 594 beschrieben, hergestellt werden können.

Die Isolierung der 1-Brom-3- Chlor-1,2-diaryl-2-propanole I aus dem rohen Reaktionsgemisch kann in an sich bekannter Weise z.B. durch Abdampfen des Lösungsmittels und ggf. überschüssigem Brom oder durch Waschen der organischen Phase mit verdünnten Alkalilösungen und Eindampfen der organischen Phase erfolgen.

Die Diastereomerenanteile an erythro- und threo-Isomeren der 1-Brom-3-chlor-1,2-diaryl-2-propanole I können z.B. durch HPLC (Hochdruckflüssigkeitschromatographie) oder durch $^1$H-NMR-Untersuchungsmethoden, ggf. unter Verwendung der reinen Isomeren als Vergleichsmittel, bestimmt werden. Im $^1$H-NMR-Spektrum wird ein charakteristisches Signal durch die -CH-Br-Gruppe (Singulett) erzeugt, das im Fall der erythro-Konfiguration üblicherweise bei etwas tieferem Feld auftritt. Die Zuordnung der relativen Konfiguration kann zum Beispiel auch durch Röntgenstrukturanalyse erfolgen.

Im Hinblick auf die biologische Wirksamkeit der Endprodukte III haben die Substituenten $R^1$ und $R^2$ im erfindungsgemäßen Zwischenprodukt I bevorzugt die folgende Bedeutung:
Wasserstoff;
Halogen wie Fluor, Chlor, Brom und Jod, vorzugsweise Chlor und Fluor;
$C_1$-$C_6$-Halogenalkyl wie Difluormethyl, Trifluormethyl, Trichlormethyl, Pentafluorethyl, vorzugsweise Trifluormethyl;
$C_1$-$C_5$-Alkoxy wie Methoxy, Ethoxy, Propoxy, 1-Methylethoxy, n-Butoxy, tert.-Butoxy, 1-Methylpropoxy, 2-Methylpropoxy, vorzugsweise Methoxy, Ethoxy und Propoxy;
$C_1$-$C_5$-Halogenalkoxy wie Difluormethoxy, Trifluormethoxy, Chlordifluormethoxy, Dichlorfluormethoxy, 1-Fluorethoxy, 2-Fluorethoxy, 2,2-Difluorethoxy, 1,1,2,2-Tetrafluorethoxy, 2, 2, 2-Trifluorethoxy, 2-Chlor-1,1,2-trifluorethoxy und Pentafluorethoxy, vorzugsweise Trifluormethoxy;
tert.-Butyl;
einen aromatischen Rest, z.B. einen Phenylrest, der unsubstituiert ist oder ein bis drei Reste aus der Gruppe Halogen, $C_1$-$C_5$-Alkoxy oder $C_1$-$C_5$-Halogenalkoxy trägt, wobei diese Reste die oben für $R^1$ und $R^2$ speziell genannte Bedeutung haben können.

Die Indices n und m stehen bevorzugt für 1 oder 2, insbesondere 1.

Beispielsweise können die in der folgenden Tabelle 1 aufgeführten Substitutionsmuster in den 1-Brom-3-chlor-1,2-diaryl-2-propanolen der allgemeinen Formel I vorliegen:

Tabelle 1

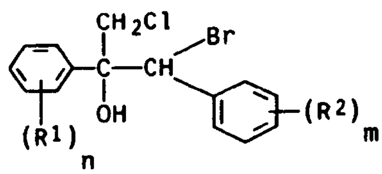

(I)

| Verbindung Nr. | $(R^1)_n$ | $(R^2)_m$ |
|---|---|---|
| 1 | 3-Cl | 3-Cl |
| 2 | 4-Cl | 2,4-diCl |
| 3 | 4-F | H |
| 4 | 4-F | 2-$CF_3$ |
| 5 | 4-F | 2-Cl |

Tabelle 1 (Forts.)

| Verbindung Nr. | $(R^1)_n$ | $(R^2)_m$ |
|---|---|---|
| 6 | 4-$H_3CO$ | 2-Cl |
| 7 | 4-Br | 2,4-diCl |
| 8 | 4-Cl | 2-$CF_3$-O- |
| 9 | 4-$C_6H_5$ | 2-Cl |
| 10 | 4-Cl-$C_6H_4$ | H |
| 11 | 4-F | 3-$C_6H_5$ |
| 12 | 4-$CF_3$-O- | 2-Cl |
| 13 | H | H |
| 14 | H | 2-$CF_3$- |
| 15 | H | 2-$CF_3$-O- |

Ausgehend von den erfindungsgemäßen 1-Brom-3-chlor-1,2-diaryl-2-propanolen I können die fungizid wirksamen Azolylmethyloxirane III vorteilhaft hergestellt werden, wobei zur Herstellung der bevorzugten cis-Azolylmethyloxirane, bei denen die Arylgruppen transoid zueinander angeordnet sind, die erythro-konfigurierten Isomeren der Formel Ia und Ib eingesetzt werden.

Demgemäß wird erfindungsgemäß ein Verfahren zur Herstellung von Azolylmethyloxiranen der Formel III

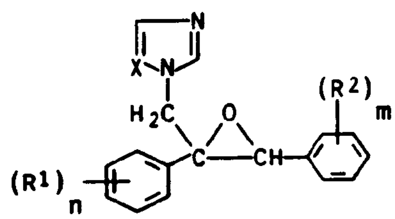

(III)

in der die Reste $(R^1)_n$ und $(R^2)_m$ die in Anspruch 1 genannte Bedeutung haben und X für CH oder N steht, zur Verfügung gestellt, das dadurch gekennzeichnet ist, daß man die 1-Brom-3-chlor-1,2-diaryl-2-propanole der Formel I mit 1,2,4-Triazol oder Imidazol in Gegenwart einer Base oder direkt mit dem 1,2,4-Triazolid oder dem Imidazolid in einem inerten Lösungsmittel umsetzt.

Die Umsetzung der 1-Brom-3-chlor-1,2-diaryl-2-propanole I mit 1,2,4-Triazol oder Imidazol in Gegenwart einer Base verläuft überraschenderweise regioselektiv zu den Azolylmethyloxiranen der Formel III, wobei praktisch ausschließlich an der Chlormethyl-Seitenkette der Verbindung I substituiert wird und sich durch inverse, intramolekulare Substitution der Hydroxylgruppe der Oxiranring in situ ausbildet. Allgemein verläuft die Oxiranbildung etwas schneller als die Substitution durch das Triazolid bzw. Imidazolid, so daß je nach Reaktionsführung auch das zumindest teilweise intermediär auftretende Chlormethyloxiran IV

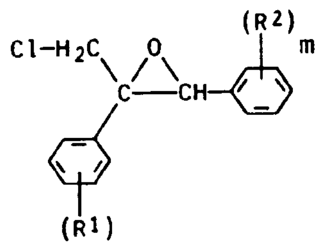

(IV)

isoliert und durch Folgeumsetzung mit dem 1,2,4-Triazolid oder dem Imidazolid in das Endprodukt III überführt werden kann. Vorteilhaft wird aber direkt bis zum Azolylmethyloxiran III ohne Isolierung von IV umgesetzt.

Die Substitutionsreaktion kann durch folgende Reaktionsgleichungen, worin Me für ein Metallatom steht, dargestellt werden:

a) I +
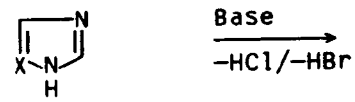

III

b) I +
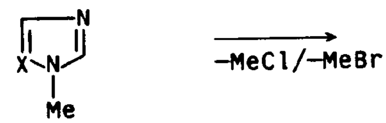

III

Die Reaktion a) erfolgt gegebenenfalls in Gegenwart eines Lösungs- oder Verdünnungsmittels, unter Zusatz von 2 bis 3 Moläquivalenten einer anorganischen oder organischen Base und gegebenenfalls unter Zusatz eines Reaktionsbeschleunigers.

Zu den bevorzugten Lösungs- oder Verdünnungsmitteln gehören Ketone wie Aceton, Methylethylketon oder Cyclohexanon, Nitrile wie Acetonitril, Ester wie Essigsäureethylester, Ether wie Diethylether, Tetrahydrofuran oder Dioxan, Sulfoxide wie Dimethylsulfoxid, Amide wie Dimethylformamid, Dimethylacetamid oder N-Methylpyrrolidon, ferner Sulfolan oder entsprechende Gemische.

Geeignete Basen, die gegebenenfalls auch als säurebindende Mittel bei der Reaktion verwendet werden können, sind beispielsweise Alkalihydroxide wie Lithium-, Natrium- oder Kaliumhydroxid; Alkalicarbonate wie Natrium- oder Kaliumcarbonat oder Natrium- und Kaliumhydrogencarbonat, Überschüsse an 1,2,4-Triazol. Es können aber auch andere übliche Basen wie Alkalihydride, Alkaliamide und insbesondere Alkoholate, z.B. Alkalialkoholate wie Natriummethylat, Natrium- oder Kalium-tert.-butylat verwendet werden.

Als Reaktionsbeschleuniger kommen vorzugsweise Metallhalogenide wie Natriumiodid oder Kaliumiodid, quaternäre Ammoniumsalze wie Tetrabutylammoniumchlorid, -bromid oder -iodid, Benzyl-tri-ethylammoniumchlorid oder -bromid oder Kronenether wie 12-Krone-4, 15-Krone-5, 18-Krone-6, Dibenzo-18-Krone-6 oder Dicyclohexano-18-Krone-6 in Frage.

Die Umsetzung wird im allgemeinen bei Temperaturen zwischen 20 und 110°C drucklos oder unter Druck, kontinuierlich oder diskontinuierlich durchgeführt.

Ist Me ein Metallatom wird die Reaktion gegebenenfalls in Gegenwart eines Lösungs- oder Verdünnungsmittels und gegebenenfalls unter Zusatz einer starken anorganischen oder organischen Base durchgeführt. Metallatome sind z.B. Natrium und Kalium.

Geeignete Basen, die gegebenenfalls auch als säurebindende Mittel bei der Reaktion verwendet werden können, sind beispielsweise Alkalihydride wie Lithium-, Natrium- und Kaliumhydrid, Alkaliamide wie Natrium- und Kaliumamid, ferner Lithium-, Natrium- oder Kalium-triphenylmethyl, Naphthalinlithium, -natrium oder -kalium und insbesondere Alkoholate wie Natrium- oder Kalium-tert.-butylat oder Natriummethylat.

Für die Reaktion b) kommen als Verdünnungsmittel polare organische Lösungsmittel wie Nitrile, z.B. Acetonitril, Sulfoxide, z.B. Dimethylsulfoxid, Formamide, z.B. Dimethylformamid, Ketone, z.B. Aceton, Ether, z.B. Diethylether, Tetrahydrofuran und insbesondere Chlorkohlenwasserstoffe, z.B. Methylenchlorid und Chloroform, in Frage.

Die Umsetzung wird im allgemeinen zwischen 0 und 100°C, vorzugsweise bei 50 bis 80°C durchgeführt. Im allgemeinen wird das Triazol in einer Menge von 1 bis 3, insbesondere 1 bis 1,5 Moläquivalenten, bezogen auf das Chlorhydrin II, verwendet. Die Basenmenge liegt üblicherweise bei 2 bis 5, insbesondere 2 bis 2,5 Moläquivalenten, bezogen auf das Chlorhydrin II.

Bei Verwendung von Triazoliden, z.B. Natriumtriazolid, die sowohl als Base wie auch als Reaktionspartner dienen, können z.B. 2 bis 3 Moläquivalente, bezogen auf II, eingesetzt werden.

Gemäß einer vorteilhaften Ausführungsform des Verfahrens werden ausgehend von den erythro-konfigurierten 1-Brom-3-chlor-1,2-diaryl-2-propanolen Ia und Ib regio- und stereoselektiv die cis- oder Z-Azolylmethyloxirane IIIa

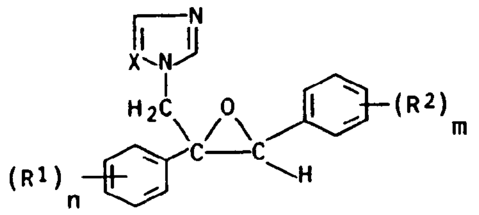

(IIIa)

hergestellt.

Aus den geringen Anteilen an threo-konfigurierten 1-Brom-3-chlor-1,2-diaryl-2-propanolen I im Reaktionsgemisch, werden entsprechend die trans- bzw. E-Azolylmethyloxirane III erhalten.

Die einzelnen Syntheseschritte sind in den nachfolgenden Versuchsbeispielen erläutert.

Beispiel 1

Herstellung der Ausgangsstoffe II durch Grignard-Reaktion

1-Chlor-3-(2-chlorphenyl)-2-(4-fluorphenyl)-2-propanol

Bei Raumtemperatur werden 30 g (1,25 mol) Magnesiumspäne in 100 ml Diethylether vorgelegt und innerhalb von 4 bis 5 Minuten 17g (0,1 mol) 2-Chlorbenzylchlorid zugetropft. Nach dem Anspringen der Grignard-Reaktion werden weitere 153 g (0,95 mol) 2-Chlorbenzylchlorid in 800 ml Diethylether bei Rückflußtemperatur innerhalb von ca. einer Stunde zugetropft. Nach weiteren 30 Minuten, kühlt man ab und tropft die Grignard-Lösung bei 0 bis 2°C zu 157 g (0,91 mol) 4-Fluor-ω-chloracetophenon in 250 ml Toluol und rührt anschließend noch 1 bis 2 Stunden bei 0°C nach. Das Reaktionsgemisch wird wie üblich aufgearbeitet.

Beispiel 2

Herstellung der 1-Brom-3-chlor-1,2-diaryl-2-propanole I mittels Photobromierung

1-Brom-3-Chlor-1-(2-chlorphenyl)-2-(4-fluorphenyl)-2-propanol (Verbindung Nr. 5 aus Tabelle 1)

4,0 g (13,4 mmol) 1-Chlor-3-(2-chlorphenyl)-2-(4-fluorphenyl)-2-propanol werden mit 2,3 g (8,0 mmol) 1,3-Dibrom-5,5-dimethylhydantoin bei 35°C in 80 ml Tetrachlorkohlenstoff unter gutem Rühren und Bestrahlen mit einer Mischlichtlampe (Osram HWL 235 V/500 W) photobromiert. Nach Absaugen von Hydantoin und extrahieren mit verdünnter Natriumcarbonatlösung und Wasser werden nach üblicher Aufarbeitung 5,1 g Rohprodukt als Öl erhalten, das für die Folgestufe eingesetzt wird. Aus dem Signal der -CH-Br-Gruppe im $^1$H-NMR-Spektrum das für die erythro-konfigurierte Verbindung bei 6,4 ppm und für die threo-konfigurierte Verbindung bei 6,2 ppm (jeweils in $CDCl_3$ gemessen) erscheint, wird ein Isomerenverhältnis erythro:threo von 4:1 ermittelt. Das Erythro-Isomer ist ferner durch ein Doppelduplett bei 4, 1 ppm charakterisiert.

Beispiel 3

Umsetzung der 1-Brom-3-chlor-1,2-diaryl-2-propanole I zu den Azolylmethyloxiranen III

cis-2-(1,2,4-Triazol-1-ylmethyl)-3-(2-chlorphenyl)2-(4-fluor-phenyl)oxiran

3,9 g (10,4 mmol) des oben hergestellten rohen 1-Brom-3-chlor-1-(2-chlorphenyl)2-(4-fluorphenyl)-2-propanols werden in 15 ml Dimethylformamid mit 2,4 g (26,4 mmol) Natrium-1,2,4-Triazolid 4 Stunden bei 75°C gerührt. Anschließend neutralisiert man mit etwas Eisessig und verdünnt das Reaktionsgemisch mit ca. 15 bis 20 ml Wasser, wobei unter gutem Rühren cis-2-(1,2,4-Triazol-1-ylmethyl)-3-(2-chlorphenyl)-2-(4-fluor-phenyl)-oxiran als rohes Kristallisat ausfällt. Absaugen, Nachwaschen mit DMF/Wasser (1:1) und Auskochen des Rückstandes mit etwas n-Pentan und Cyclohexan liefert nach dem Trocknen 1,85 g Wertprodukt als farbloses Kristallisat (ca. 58 % Ausbeute).

Beispiel 4

Herstellung der 1-Brom-3-chlor-1,2-diaryl-2-propanole I mittels Bromierung in Gegenwart eines Radikalstarters

1-Brom-3-chlor-1-(2-chlorphenyl)-2-(4-fluorphenyl)-2-propanol

4,0 g (13,4 mmol) 1-Chlor-3-(2-chlorphenyl)-2-(4-fluorphenyl)-2-propanol werden in 40 ml Dichlorethan unter trockenem Stickstoff oder Kohlendioxid mit 2,29 g (8,0 mmol) 1,3-Dibrom-5,5-dimethylhydantoin und 0,2 g Azo-bis isobutyronitril (Phorophor N) versetzt und 10 Stunden bei 72 bis 74°C gerührt, wobei man nach 5 Stunden erneut mit 0,2 g Phorophor N versetzt. Die Aufarbeitung erfolgt wie bei der Photobromierung unter Beispiel 2 angegeben. Die Ausbeute an noch leicht lösungsmittelfeuchtem Rohprodukt beträgt 6,2 g, das Isomerenverhältnis erythro:threo liegt bei ca. 3:1.

## Patentansprüche

**1.** 1-Brom-3-chlor-1,2-diaryl-2-propanole der allgemeinen Formel I

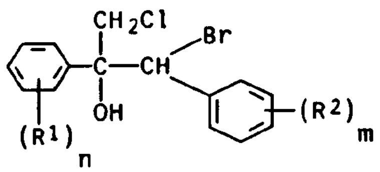

(I)

in welcher n und m für 1, 2 oder 3 stehen und die Substituenten $R^1$ und $R^2$ unabhängig voneinander die folgende Bedeutung haben:
Wasserstoff, Halogen, eine $C_1$-$C_6$-Halogenalkylgruppe, eine $C_1$-$C_5$-Alkoxygruppe, eine $C_1$-$C_5$-Halogenalkoxygruppe, t-Butyl oder einen aromatischen Rest, der unsubstituiert oder ein- bis dreifach durch Halogen, eine $C_1$-$C_5$-Alkoxygruppe, eine $C_1$-$C_5$-Halogenalkoxygruppe oder t-Butyl substituiert ist.

**2.** Erythro-1-Brom-3-chlor-1,2-diaryl-2-propanole der Formel I gemäß Anspruch 1, in der $(R^1)_n$ Wasserstoff oder 4-Halogen und $(R^2)_m$ 2-Trifluormethyl oder 2-Halogen bedeuten.

**3.** Erythro-1-Brom-3-chlor-1,2-diaryl-2-propanole der Formel I gemäß den Ansprüchen 1 und 2, in der $R^1$ 4-Fluor und $R^2$ 2-Trifluormethyl, 2-Chlor oder 2-Brom bedeuten.

**4.** Verfahren zur selektiven Herstellung von erythro-1-Brom-3-chlor-1,2-diaryl-2-propanolen der Formel I

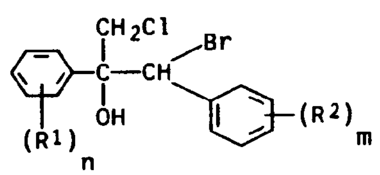

(I),

in welcher n und m für 1, 2 oder 3 stehen und die Substituenten $R^1$ und $R^2$ unabhängig voneinander die folgende Bedeutung haben:
Wasserstoff, Halogen, eine $C_1$-$C_6$-Halogenalkylgruppe, eine $C_1$-$C_5$-Alkoxygruppe, eine $C_1$-$C_5$-Halogenalkoxygruppe, t-Butyl oder einen aromatischen Rest, der unsubstituiert oder ein- bis dreifach durch Halogen, eine $C_1$-$C_5$-Alkoxygruppe, eine $C_1$-$C_5$-Halogenalkoxygruppe oder t-Butyl substituiert ist, dadurch gekennzeichnet, daß man Chlorhydrine der allgemeinen Formel II,

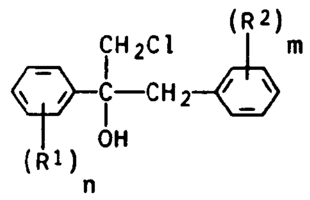

(II),

in der die Reste die o. g. Bedeutung haben, in einem inerten aprotischen Lösungsmittel durch radikalische Bromierung in die Verbindungen der Formel I überführt.

5. Verfahren nach Anspruch 4, dadurch gekennzeichnet, daß man als Bromierungsreagenzien Brom, N-Bromsuccinimid oder N,N'-Dibrom-dimethylhydantoin verwendet und die Bromierung unter gleichzeitigem Bestrahlen mit einer Photolampe oder unter Verwendung eines Radikalstarters durchführt.

6. Verfahren nach Anspruch 4, dadurch gekennzeichnet, daß man 1 bis 5 Moläquivalente der Bromierungsreagenzien, je Mol Chlorhydrin II, verwendet.

7. Verfahren nach Anspruch 4, dadurch gekennzeichnet, daß man als Lösungsmittel für die Bromierung halogenierte aliphatische oder aromatische Kohlenwasserstoffe verwendet.

8. Verfahren nach Anspruch 4, dadurch gekennzeichnet, daß man die Bromierung bei Temperaturen von 0 bis 100°C durchführt.

9. Verfahren zur Herstellung von Azolylmethyloxiranen der Formel III

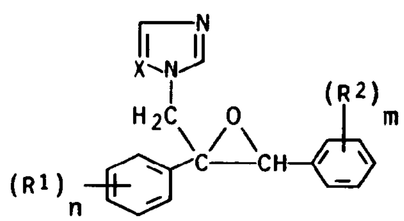

(III),

in der die Reste $(R^1)_n$ und $(R^2)_m$ die in Anspruch 1 genannte Bedeutung haben und X für CH oder N steht, dadurch gekennzeichnet, daß man die 1-Brom-3-chlor-1,2-diaryl-2-propanole der Formel I gemäß Anspruch 1 mit 1,2,4-Triazol oder Imidazol in Gegenwart einer Base oder direkt mit dem 1,2,4-Triazolid oder dem Imidazolid in einem inerten Lösungsmittel umsetzt.

10. Verfahren nach Anspruch 9, dadurch gekennzeichnet, daß man ausgehend von den erythro-1-Brom-3-chlor-1,2-diaryl-2-propanolen der Formel I regio- und stereoselektiv die Z-Azolylmethyloxirane der Formel III a

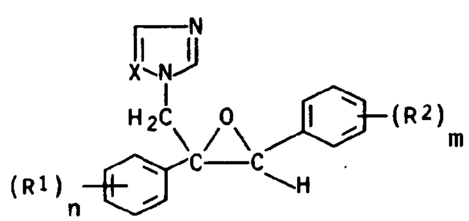

(IIIa)

herstellt.

## Claims

1. A 1-bromo-3-chloro-1,2-diaryl-2-propanol of the formula I

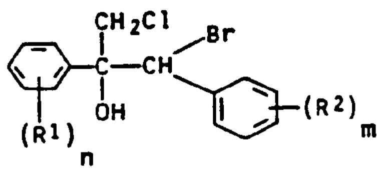

(I),

where n and m are each 1, 2 or 3, and $R^1$ nd $R^2$ are each, independently of one another, hydrogen, halogen, $C_1$-$C_6$-haloalkyl, $C_1$-$C_5$-alkoxy, $C_1$-$C_5$-haloalkoxy, t-butyl or an aromatic radical which is unsubstituted or substituted once to three times by halogen, $C_1$-$C_5$-alkoxy, $C_1$-$C_5$-haloalkoxy or t-butyl.

2. An erythro-1-bromo-3-chloro-1,2-diaryl-2-propanol of the formula I as claimed in claim 1, where $(R^1)_n$ is hydrogen or 4-halo and $(R^2)_m$ is 2-trifluoromethyl or 2-halo.

3. An erythro-1-bromo-3-chloro-1,2-diaryl-2-propanol of the formula I as claimed in either of claims 1 and 2, where $R^1$ is 4-fluoro and $R^2$ is 2-trifluoromethyl, 2-chloro or 2-bromo.

4. A process for the selective preparation of an erythro-1-bromo-3-chloro-1,2-diaryl-2-propanol of the formula I

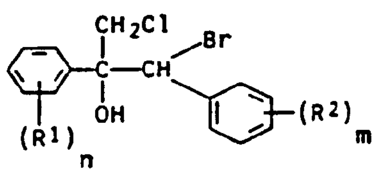

(I),

where n and m are each 1, 2 or 3, and $R^1$ and $R^2$ are each, independently of one another, hydrogen, halogen, $C_1$-$C_6$-haloalkyl, $C_1$-$C_5$-alkoxy, $C_1$-$C_5$-haloalkoxy, t-butyl or an aromatic radical which is unsubstituted or substituted once to three times by halogen, $C_1$-$C_5$-alkoxy, $C_1$-$C_5$-haloalkoxy or t-butyl, which comprises converting a chlorohydrin of the formula II

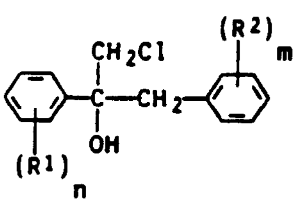

(II),

where the radicals have the abovementioned meanings, in an inert aprotic solvent by free radical bromination into the compound of the formula I.

5. A process as claimed in claim 4, wherein bromine, N-bromosuccinimide or 1,3-dibromo-5,5-dimethylhydantoin is used as brominating reagent, and the bromination is carried out with irradiation by light or using a radical initiator.

6. A process as claimed in claim 4, wherein 1 to 5 mole equivalents of the brominating reagent per mole of chlorohydrin II are used.

7. A process as claimed in claim 4, wherein halogenated aliphatic or aromatic hydrocarbons are used as solvents for the bromination.

8. A process as claimed in claim 4, wherein the bromination is carried out at from 0 to 100°C.

**9.** A process for the preparation of an azolylmethyloxirane of the formula III

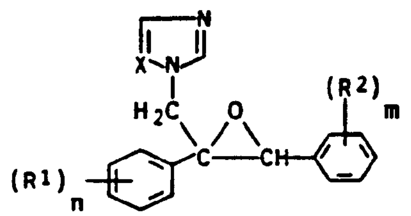

(III),

where $(R^1)_n$ and $(R^2)_m$ have the meanings specified in claim 1, and X is CH or N, which comprises reacting a 1-bromo-3-chloro-1,2-diaryl-2-propanol of the formula I as claimed in claim 1 with 1,2,4-triazole or imidazole in the presence of a base or directly with the 1,2,4-triazolide or the imidazolide in an inert solvent.

**10.** A process as claimed in claim 9, wherein a Z-azolylmethyloxirane of the formula III a

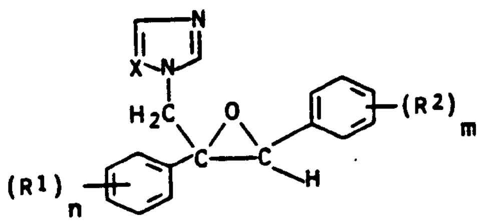

(IIIa)

is prepared regio- and stereoselectively starting from an erythro-1-bromo-3-chloro-1,2-diaryl-2-propanol of the formula I.

## Revendications

**1.** 1-bromo-3-chloro-1,2-diaryl-2-propanols de formule générale I

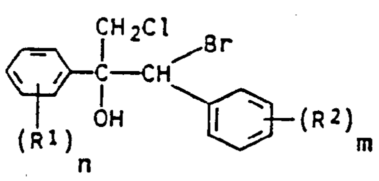

(I)

dans laquelle n et m sont mis pour 1, 2 ou 3 et les substituants $R^1$ et $R^2$ ont, indépendamment l'un de l'autre la signification suivante :
hydrogène, halogène, un groupe halogéno-alkyle en $C_1$-$C_6$, un groupe oxy, un groupe halogéno-alcoxy en $C_1$-$C_5$, t-butyle ou un reste aromatique, qui n'est pas substitué ou est substitué une à trois fois par halogène, un groupe alcoxy en $C_1$-$C_5$, un groupe halogénoalcoxy en $C_1$-$C_5$ ou t-butyle.

**2.** Erythro-1-bromo-3-chloro-1,2-diaryl-2-propanols de formule générale I dans laquelle $(R^1)_n$ représente hydrogène ou 4-halogène et $(R^2)_m$ représente 2-trifluorométhyle ou 2-halogène.

**3.** Erythro-1-bromo-3-chloro-1,2-diaryl-2-propanols de formule générale I selon les revendications 1 et 2, dans laquelle $R^1$ représente 4-fluor et $R^2$ 2-trifluorométhyle, 2-chlore ou 2-brome.

**4.** Procédé de préparation sélective de érythro-1-bromo-3-chloro-1,2-diaryl-2-propanols de formule générale I

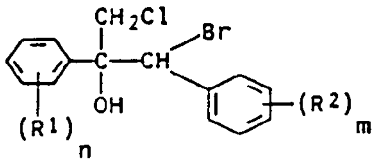

(I),

dans laquelle n et m sont mis pour 1, 2 ou 3 et les substituants $R^1$ et $R^2$ ont, indépendamment l'un de l'autre la signification suivante:
hydrogène, halogène, un groupe halogéno-alkyle en $C_1$-$C_6$, un groupe alcoxy en $C_1$-$C_5$, un groupe halogéno-alcoxy en $C_1$-$C_5$, t-butyle ou un reste aromatique, qui n'est pas substitué ou est substitué une à trois fois par halogène, un groupe alcoxy en $C_1$-$C_5$, un groupe halogénoalcoxy en $C_1$-$C_5$ ou t-butyle,
caractérisé par le fait qu'on transforme en les composés de formule I, par bromation engendrant des radicaux dans un solvant aprotique inerte, de la chlorhydrine de la formule générale II

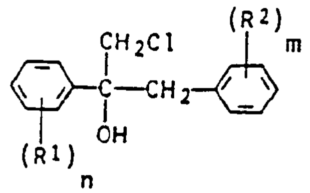

(II),

dans laquelle les restes ont la signification sus-indiquée.

5. Procédé selon la revendication 4, caractérisé par le fait que l'on utilise, comme agent de bromation, brome, N-bromosuccinimide ou N,N'-dibromo-diméthylhydantoine et qu'on effectue la bromation sous irradiation simultanée avec une lampe photo ou en utilisant un initiateur de radicaux.

6. Procédé selon la revendication 4, caractérisé par le fait que l'on utilise 1 à 5 équivalents molaires de l'agent de bromation par mole de chlorhydrine II.

7. Procédé selon la revendication 4, caractérisé par le fait que l'on utilise, comme solvant pour la bromation, des hydrocarbures halogènes aliphatiques ou aromatiques.

8. Procédé selon la revendication 4, caractérisé par le fait que l'on effectue la bromation à des températures de 0 à 100°C.

9. Procédé de préparation d'azolylméthyloxirannes de la formule III

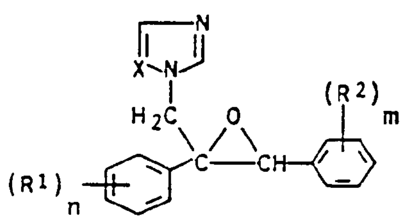

(III),

dans laquelle les restes $(R^1)_n$ et $(R^2)_m$ ont la signification indiquée dans la revendication 1 et X est mis pour CH ou N, caractérisé par le fait que l'on fait réagir dans un solvant inerte les 1-bromo-3-chloro-1,2-diaryl-2-propanols de formule I selon la revendication 1 avec du 1,2,4-triazole ou imidazole en présence d'une base ou directement avec le 1,2,4-triazole ou l'imidazole.

10. Procédé selon la revendication 9, caractérisé par le fait que, en partant des érythro-1-bromo-3-chloro-1,2-diaryl-2-propanols, on prépare regio- et stéréosélectivement les Z-azolylméthyloxirannes de la formule IIIa

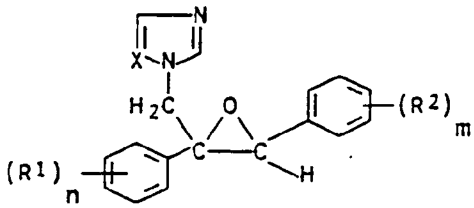

(IIIa)